# EUROPEAN PATENT SPECIFICATION

(11) **EP 0 522 470 B1**
(45) Date of publication and mention of the grant of the patent: **22.05.1996**
(21) Application number: 92111339.5
(22) Date of filing: 03.07.1992
(51) Int. Cl.: C12P 7/64, C12N 5/00, A23K 1/00

(54) **Triglyceride-containing dry cell fragments and method of preparing them**
Triglyceride enthaltende trockene Zellfragmente und Verfahren zu deren Herstellung
Fragments secs de cellules contenant des triglycérides et procédé de leur production

(30) Priority: 11.07.1991 JP 196140/91; 01.05.1992 JP 137590/92
(43) Date of publication of application: 13.01.1993
(73) Proprietor: IDEMITSU PETROCHEMICAL CO., LTD., Minato-ku, Tokyo (JP)
(72) Inventor: Sano, Toshio, c/o Idemitsu Petrochem. Co., LTD., Sodegaura-shi, Chiba-ken (JP); Touda, Masahiko, c/o Idemitsu Petrochem. Co., LTD, Sodegaura-shi, Chiba-ken (JP); Aoyama, Tomoya, c/o Idemitsu Petrochem. Co., LTD., Chiyoda-ku, Tokyo (JP)
(74) Representative: Türk, Dietmar, Dr. rer. nat.

(56) References cited:
- EP-A- 0 252 716
- EP-A- 0 269 351
- WO-A-86/04354
- DE-A- 3 201 427

## Description

### FIELD OF THE INVENTION

The present invention relates to triglyceride-containing dry cell fragments useful as feed for stock farming and as pet food and to a method of preparing them.

The present invention also relates to a feed composition containing triglyceride-containing dry cell fragments.

The present invention further relates to a method of extracting and recovering lipids (triglycerides) from triglyceride -containing microbial cells extremely efficiently and safely.

### BACKGROUND OF THE INVENTION

As microorganisms having the ability of producing polyunsaturated fatty acids such as γ -linolenic acid (GLA), dihomo-γ -linolenic acid (DGLA), arachidonic acid (AA), eicosapentaenoic acid (EPA) and others, there are mold and yeast; and it has already been known to use such microorganisms as feed for animals (for example, JP-A 61-149054, 63-98355 and 1-215245 - the term "JP-A" as used herein means an "unexamined published Japanese patent application - and British Patent 2114442).

Since lipids containing polyunsaturated fatty acids such as triglycerides and others are accumulated in the inside of microbial cells, they must be extracted out from the cells directly or by mechanically or enzymatically breaking the cell walls, in order to utilize them.

As such a technique, for example, there is known a breaking and extracting method of suspending microbial cells in ethanol, breaking them therein, removing ethanol by filtration and/or centrifugation, adding an extracting solvent thereto to again suspend the thus broken cells in the solvent, and extracting the intended polyunsaturated fatty acids from the suspension (JP-A 61-170397, 61-227790, 62-44170, 62-179598, etc.).

In accordance with the method, however, two different solvents are used and separation of solid matter (cell debris) from the organic solvent used is necessary. Thus, the method is defective as the operation thereof is complicated. In addition, since the cells are broken in an organic solvent, there are other problems of the danger of fire and the necessity of large-scaled equipments.

Moreover, where polyunsaturated fatty acids are brought into contact with oxygen in air, they easily react with it to form peroxides, whereby the content of the polyunsaturated fatty acids lowers during storage or during heat-treatment for granulation. Further, some offensive odor would be generated from the peroxides and decomposed products of them, which causes still another problem that the stored or heat-treated feed of containing polyunsaturated fatty acids is disliked by animals.

### SUMMARY OF THE INVENTION

One object of the present invention is to overcome the above-mentioned drawbacks in utilizing microbial cells containing triglycerides including polyunsaturated fatty acids as feed for stock farming and as pet food and to provide a method of preventing lowering of the content of polyunsaturated fatty acids in the cells in heat-treatment of them.

Another object of the present invention is to provide a method of extracting and recovering lipids or, that is, triglycerides from microbial cells extremely efficiently and safely.

First, the present invention provides, for the purpose of attaining the above-mentioned objects, a method of preparing triglyceride-containing dry cell fragments in which the decrement of the content of polyunsaturated fatty acids after heat-treatment at 150° C for 2 minutes is not more than 10 % by weight; the method being characterized in that triglyceride-containing microbial cells are broken, while they are dispersed in water, and then dewatered.

Second, the present invention provides triglyceride-containing dry cell fragments in which the decrement of the content of polyunsaturated fatty acids after heat-treatment at 150° C for 2 minutes is not more than 10 % by weight; the cell fragments being obtained by breaking triglyceride-containing microbial cells, while the cells are dispersed in water, followed by dewatering them.

Thirdly, the present invention provides triglyceride-containing dry cell fragments in which the decrement of the content of polyunsaturated fatty acids after heat-treatment at 150° C for 2 minutes is not more than 10 % by weight.

Fourthly, the present invention provides a feed composition containing the cell fragments of the third invention.

Last and fifthly, the present invention provides a method of recovering triglycerides from triglyceride-containing microbial cells; which is characterized in that triglyceride-containing microbial cells are broken, while they are dispersed in water, then dewatered and extracted with a solvent.

### BRIEF EXPLANATION OF THE DRAWINGS

Fig. 1 shows an outline of a flow sheet of a process of producing dog food.

Fig. 2 is an explanatory view of showing the column used in Example 1, where a is cells, b is absorbent cotton and c is a cock valve.

### DETAILED DESCRIPTION OF THE INVENTION

Triglyceride-containing microbial cells to be used in the present invention are ones to be obtained by cultivating microorganisms having the ability of producing triglycerides by ordinary methods.

As microorganisms having the ability of producing triglycerides as referred to herein, there are various mold, yeast and algae, as mentioned above.

For instance, as microorganisms having the ability of producing γ-linolenic acid-containing lipids, there are mentioned microorganisms of belonging to the genus Mortierella as described in JP-A 60-168391; microorganisms of belonging to the genus Mucor as described in JP-A 63-283589; and microorganisms of belonging to the genus Rhizopus as described in JP-A 63-133994, etc.

As microorganisms having the ability of producing dihomo-γ -linolenic acid-containing lipids and arachidonic acid-containing lipids, for example, there are mentioned microcrganisms of belonging to the genus Mortierella as described in JP-A 63-14696, 63-12290 and 63-44891; and microorganisms of belonging to the genus Conidiobolus as described in JP-A 64-47384, 64-47385 and 63-102688, etc.

More specifically, as microorganisms of belonging to the genus Mortierella, for example, there are mentioned Mortierella isabellina IFO 7824, Mortierella ramaniana var. angrispora IFO 8187, etc.

As microorganisms of belong to the genus Mucor, there are mentioned, for example, Mucor circinelloides HUT 1121 (BP-3883), Mucor javanicus HUT 1162 (FERM BP-3884), etc.

As microorganisms of belong to the genus Conidiobolus, there are mentioned, for example, Conidiobolus heterosporus ATCC 12941, Conidiobolus nanodes CBS 183/62 (ATCC 14651), Conidiobolus lamprauges ATCC 12585, etc.

All of them have the ability of producing lipids(triglycerides), and they may accumulate a large amount of lipids (triglycerides) in the inside of the cells of themselves.

Cultivation of such microorganisms may be effected by ordinary methods. For instance, media to be used for cultivating the above-mentioned microorganisms may be any ordinary ones in which the microorganisms may well grow to produce the intended lipids(triglycerides). They contain, for example, glucose, starch and the like carbon sources, along with ammonium sulfate, urea as well as defatted soybean powder, defatted rice bran and the like organic nitrogen sources. In addition, they may further contain, if necessary, phosphates and metal salts such as magnesium salts, manganese salts and calcium salts, as well as vitamins and amino acids.

The cultivating conditions of pH, temperature and time may suitably be controlled in such a way that the intended lipids(triglycerides) may satisfactorily be produced and accumulated in the microorganisms cultivated.

In general, since triglycerides are accumulated in the inside of microbial cells, the cultivated cells are harvested from the fermentation broth by filtration, centrifugation or the like solid-liquid separation.

In accordance with the method of the present invention, the harvested cells are broken while they are dispersed in water. Precisely, the harvested cells are disintegrated while they are again dispersed and suspended in water or after they have been again dispersed and suspended in water.

When the cells are dispersed and suspended in water, the concentration of the cells is preferably higher. However, in consideration of the fluidity of the cell dispersion or suspension during transportation of it with a pump, the concentration of the cells is adjusted to be from 5 to 18 % by weight, desirably from 8 to 15 % by weight. In order to prevent clogging of apparatus in the step of breaking the cells with a cell-breaker or the like, it is desired that the cells are previously roughly milled with a dispersing machine such as Disper Mill (trade name) or the like. Where the cell concentration in the culture liquid is sufficiently high or the culture liquid is free from any inconvenient side products which would creep into the post-steps or into the products to cause some troubles of them, the cells may directly be fed into a breaker, without being isolated.

In general, breakage of the cells is effected mechanically. For instance, the cells are broken with a French press, an ultrasonic homogenizer or the like; or they are homogenized in the presence of glass beads; or they are milled with a ball mill.

For continuously carrying out dispersion, suspension and breakage of the cells, a high-pressure homogenizer type device which is generally used for emulsification or homogenization of a fine powder suspension may also be used effectively. In addition, DYNO-MILL and PERL MILL (both are trade names) may also be used.

If desired, the cells may also be dispersed in a hot water, in place of a cold water, to form a cell suspension having a temperature of from 50 to 80° C so as to inactivate the lipase of the microorganisms. Accordingly, decomposition of triglycerides due to lipase, which would occur during the process of from breakage to drying, could be inhibited so that an increase of the acid value of the lipids to be in the product could thereby be inhibited.

Next, the thus broken cell fragments are dewatered. In the case, dewatering may be effected in such a way that the water content in the cell fragments may be 10 % by weight or less, preferably 5 % by weight or less, and it is not always necessary to effect the dewatering to the water content of being 0 % or to the complete dryness of the cell fragments. The dewatering may be carried out by the use of, for example, a freeze-dryer, vacuum dryer, pneumatic dryer, spray dryer, puddle dryer, drum dryer or the like.

The triglyceride-containing dry cell fragments of the present invention, which are obtained in the manner mentioned above, have a decrement of a polyunsaturated fatty acids content of not more than 10 % by weight after heat-treatment thereof at 150 ° C for 2 minutes. As opposed to them, those having a decrement of a polyunsaturated fatty acids content of 10 % by weight or more are unstable and are easily deteriorated so that they are not suitable as feed. The cell fragments are desired to have a peroxide value (POV) of not more than 10. If they have POV of more than 10, the feed containing them would be disliked by animals.

Where the triglyceride-containing dry cell fragments of the present invention having the above-mentioned properties are used as feed, they may be blended with ordinary feed base materials, such as grains, cereals, rice bran and lees, vitamins, minerals and the like. The proportion of the triglyceride-containing dry cell fragments to the feed base materials is not specifically defined but may suitably be determined in consideration of the use and object of the feed. In general, the dry cell fragments are incorporated in a proportion of from 0.001 to 100 % by weight, preferably from 0.01 to 20 % by weight.

The feed composition containing the triglyceride-containing dry cell fragments of the present invention is prepared by blending determined amounts of the necessary components, kneading them optionally under heat, and forming the mixture into desired shapes of powder, pellets or tablets.

In order to recover triglycerides in accordance with the present invention, the cell fragments as dewatered in the manner as mentioned above are extracted with a solvent. Precisely, after dewatered, the cell fragments are extracted with a solvent to recover the triglycerides therefrom. As solvents usable for extraction, there are mentioned, for example, n-hexane, ethanol, acetone, methyl ethyl ketone, cyclohexanone, diethyl ether, ethyl acetate and others. Especially preferred is n-hexane from the viewpoint of the extraction yield and the applicability of the recovered triglycerides to food.

The extraction method with a solvent is not specifically defined. In particular, preferred is extraction with a column. For extraction and recovery of triglycerides with a column, for example, the dewatered cell fragments are filled in a cylindrical column and an extraction solvent is introduced thereto from the top, whereupon the solvent flowing out from the bottom of the column is collected and condensed to recover the intended triglycerides. In this way, the amount of the solvent to be used may be reduced. In order to prevent leakage of solid matter (cell fragments) from the bottom of the column, it is desired that a wire gauze of a suitable size is provided in the bottom of the column or that the bottom of the column is filled with a filter aid such as diatomaceous earth or the like.

The amount of the solvent to be used for the extraction is preferably from 2 to 12 liters per kg of the cell fragments to be treated; and it is more preferably from 3 to 5 liters per the same in consideration of the extraction yield and the economic conditions.

The solvent as flown out from the bottom of the column is distilled out by ordinary condensation under reduced pressure, whereby crude lipids (crude triglycerides) are obtained. If desired, they may be purified further by ordinary methods.

The present invention will be explained in more detail by way of the following examples.

### PRODUCTION EXAMPLE 1

Mucor circinelloides HUT 1121 (FERM BP-3883) were cultivated in a medium having the composition shown in Table 1 below under the condition mentioned below, in a 300-liter fermenter for 5 days to obtain a large amount of cells. After cultivation, the fermentation broth was filtered to harvest the cells.

The thus harvested cells were uniformly dispersed and suspended in water to form a cell suspension having a cell concentration of 12 %.

**Table 1 -**

| Medium composition(for production of lipids) | |
|---|---|
| Glucose | 250 g |
| Ammonium Sulfate | 16.5 g |
| Monopotassium Phosphate | 9.0 g |
| Magnesium Sulfate 7-Hydrate | 1.0 g |
| Yeast Extract | 0.6 g |
| Ferric Sulfate 7-Hydrate | 40 mg |
| Calcium Chloride 2-Hydrate | 4.8 mg |
| Copper Sulfate 5-Hydrate | 0.8 mg |
| Zinc Sulfate 7-Hydrate | 3.5 mg |
| Manganese Chloride 4-Hydrate | 4.0 mg |
| Defoaming Agent "CC-118" | 0.6 g |
| | ( initial stage ) |
| Deionized Water | 1 liter |
| * pH 5.0 ( 30 °C ) | |

Next, using a high-pressure homogenizer (HV-OH-0.7, - 3.7S); manufactured by Izumi Food Machinery Co.), the cells in the cell suspension were continuously broken under the condition of a pressure of 700 kg/cm2 and a rate of 60 liters/hr. The thus obtained cell fragments were dewatered and dried with a double drum dryer to thereby obtain 12 kg of a dry cell fragment powder having a water content of 3.8 % by weight.

A sample as prepared by hydrolyzing the dry cell fragment powder with an alkali was analyzed by gas chromatography to contain 73.4 g/kg of γ-linolenic acid therein.

Lipids were extracted from the dry cell fragment powder with n-hexane, which were analyzed in accordance with a standard oil and fat analytical test method (established by The Japan Oil Chemist Society) to obtain a POV value thereof. As a result, they had a POV value of 0.9.

### PRODUCTION EXAMPLE 2

Mucor circinelloides HUT 1121 (FERM BP-3883) were cultivated in the same manner as in Production Example 1, and the cells were harvested from the fermentation broth by filtration.

The thus harvested cells were uniformly dispersed and suspended in water to form a suspension having a cell concentration of 12 %. Then, this was dewatered and dried with a double drum dryer to obtain about 12 kg of a dry cell powder having a water content of 4.0 % by weight.

The dry cell powder was analyzed in the same manner as in Production Example 1, and it contained 74.2 g/kg of γ-linolenic acid therein. The lipids extracted from the cell powder with n-hexane had a POV value of 0.9.

### TEST EXAMPLE 1

The stability against self-oxidation of the γ-linolenic acid-containing cells obtained in the above-mentioned Production Examples 1 and 2 was tested. Precisely, the cells obtained in each of Production Examples 1 and 2 were put in four Petri dishes each having a diameter of 9 cm and a depth of 1.5 cm, each in an amount of 5 g; and all the dishes were put in a incubator having a temperature of 40 ° C and a humidity of 50 %. At every 10 days, the dishes were taken out one by one, and the γ-linolenic acid content in the sample of each dish was measured by the same method as in Production Example 1.

The results obtained are shown in Table 2 below.

**Table 2 -**

| Variation of γ-linolenic acid content during storage of γ-linolenic acid-containing cells | | |
|---|---|---|
| Time for Storage (day) | γ-linolenic Acid Content (g/kg) | |
| | Cells of Production Example 1 | Cells of Production Example 2 |
| 0 | 73.4 | 74.2 |
| 10 | 73.0 | 68.3 |
| 20 | 72.1 | 60.1 |
| 30 | 73.1 | 53.4 |
| 40 | 71.9 | 53.1 |

As is obvious from Table 2 above, the decrement of the γ-linolenic acid content in the cells of Production Example 2 was about 30 % by weight after storage thereof for 40 days; while the γ-linolenic acid content in the cells of Production Example 1 did not vary even after storage thereof for 40 days and the cells gave almost no offensive odor after the storage.

### TEST EXAMPLE 2

Variation of the γ-linolenic acid content in the cells obtained in Production Examples 1 and 2 during the step of producing dog food of containing them was examined. Precisely, using 2.5 kg of the cells obtained in each of Production Examples 1 and 2, a dry type dog food having the formulation shown in Table 3 below was formed.

**Table 3 -**

| Formulation of Dry Type Dog Food | |
|---|---|
| Corn | 49.1 wt% |
| Gluten Feed | 19.0 wt% |
| Bone Meal | 19.0 wt% |
| Soybean Lees | 7.0 wt% |
| Animal Lipids | 2.5 wt% |
| γ-linolenic Acid-Containing Cells | 2.5 wt% |
| Mineral Mix | 0.8 wt% |
| Vitamin Mix | 0.1 wt% |
| Total | 100.0 wt% |

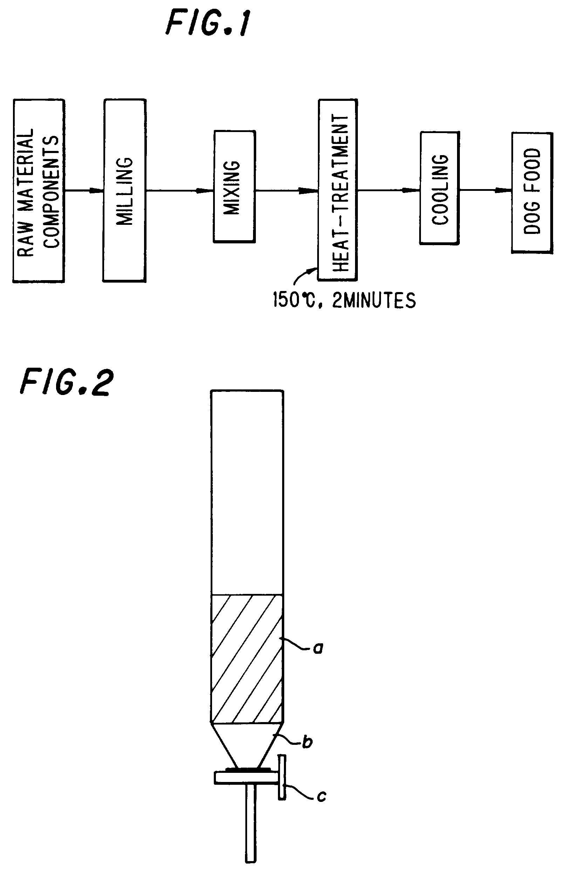

Fig. 1 shows an outline of a flow sheet of a process of producing dog food, in which the raw material components are exposed to a high temperature of 150° C in the heating step and γ-linolenic acid is deteriorated in the step.

Sampling was effected before and after the heating step, whereby the γ-linolenic acid content in each sample before and after the step was measured. The results obtained are shown in Table 4 below.

**Table 4 -**

| Variation of γ -linolenic acid content in the process of producing dry type dog food | | | |
|---|---|---|---|
| Cells of Production Example 1 | | Cells of Production Example 2 | |
| before heating | after heating | before heating | after heating |
| 1.84 | 1.80 | 1.86 | 1.49 |
| * γ-linolenic acid content : g/kg of dog food sample | | | |

As is obvious from Table 4 above, no variation of the γ -linolenic acid content was admitted before and after the heating step in the sample containing the microbial cells of Production Example 1, while the γ-linolenic acid content decreased by about 20 % by weight after the heating step in the sample containing the microbial cells of Production Example 2.

### TEST EXAMPLE 3

5 kg of each of the γ-linolenic acid containing cell preparations obtained in Production Examples 1 and 2 were put in a polyethylene bag and stored at 25° C for one month. Each of them thus stored were fed to dogs which were diagnosed as seborrheic syndrome, whereupon their taste in the cell preparations as applied thereto as well as the curing effect of their disease was examined.

There was no variation of the γ-linolenic acid content in the cell preparations of Production Examples 1 and 2 as stored for one month, but the POV value of the lipids as extracted out from them increased up to 7.5 and 114.4, respectively.

Precisely, plural groups each comprising 20 dogs with a body weight of from 5 to 6 kg, which were diagnosed as seborrheic syndrome, were used for the test. One or two g/day of the cells of each of Production Examples 1 and 2 were suspended in milk and freely fed every day to the test dogs of each group for 20 days, whereupon their taste in the cell suspension as fed thereto as well as the curing effect of their disease was examined with the naked eye. The results obtained are shown in Table 5 below.

**Table 5 - Dogs' taste in γ-linolenic acid-containing cells and the curing effect of dog seborrheic syndrome**

| ① Cells of Production Example 1 | | | | | | |
|---|---|---|---|---|---|---|
| Amount Fed | Appearance in Ingestion | | | Curing Effect | | |
| | Good | Normal | No Good | Good | Normal | No Good |
| 1.0 | 15 | 5 | 0 | 18 | 2 | 0 |
| 2.0 | 16 | 4 | 0 | 18 | 2 | 0 |

| ② Cells of Production Example 2 | | | | | | |
|---|---|---|---|---|---|---|
| 1.0 | 10 | 3 | 7 | 9 | 5 | 6 |
| 2.0 | 9 | 3 | 8 | 7 | 7 | 6 |
| * Amount Fed : as gram of cells/day/dog | | | | | | |

As is obvious from Table 5 above, the suspension of cells of Production Example 1 was totally favorably eaten by the test dogs and no test dogs denied it. In addition, the curing effect of the suspension of the cells of Production Example 1 was good. As opposed to it, about 40 % of the test dogs disliked and denied the suspension of the cells of Production Example 2, and the curing effect thereof lowered accordingly.

### REFERENTIAL EXAMPLE

In the following examples and comparative examples, the total lipids amount in the cells to be a standard for calculating the extraction yield of the all lipids therein was measured by the method mentioned below.

Mucor circinelloides HUT 1121 (FERN BP-3883) were cultured in a 30ℓ-fermenter under the condition as shown in Table 6 below, and the thus γ-linolenic acid-containing cells were harvested by filtration.

The thus harvested cells were again dispersed in water to give a 12 % suspension, a part of which was then dewatered with a double drum dryer to obtain dry cells having a water content of 4 % by weight.

100 ml of glass beads each having a diameter of 0.6 mm and 100 ml of n-hexane were added to 5 g of the dry cells, which were then homogenized with a homogenizer (Excell Auto-homogenizer DX-3 Model, manufactured by Nippon Seiki Co.) at a rotation speed of 10000 rpm for 3 minutes and thereafter filtered to remove the glass beads and the cell fragments. Next, 100 ml of n-hexane was again added to the cells, which were homogenized repeatedly two times in the same manner as above. The thus obtained filtrates were combined and concentrated under reduced pressure to obtain 1.9 g of yellow lipids. As a result, it was noted that the cells contained 38 % of lipids.

### EXAMPLE 1

Mucor circinelloides HUT 1121 (FERM BP-3883) were cultured in a 30ℓ-fermenter under the condition as shown in Table 6 below, and the thus γ-linolenic acid-containing cells were harvested by filtration.

The thus harvested cells were uniformly dispersed and suspended in water to give a 10 % suspension, which was then broken with a homogenizer (Microfludizer M110Y Model, manufactured by Microfluidize Co.) under pressure of 700 kg/cm2.

Afterwards, the broken cell fragments were freeze-dried to obtain a dry cell powder having a water content of 3.4 % by weight.

10 g of the dry cell fragment powder was filled into a glass column having an inner diameter of 20 mm, as shown in Fig. 2, and 100 ml of n-hexane was introduced thereinto from the top of the column. The n-hexane/lipids fat mixture (micelle) as flown out from the bottom of the column was recovered, and n-hexane was removed out therefrom by condensation and distillation under reduced pressure. As a result, 3.4 g of lipids were obtained. The extraction yield was 90 %.

### EXAMPLE 2

Mucor circinelloides HUT 1121 (FERM BP-3883) were cultivated in a 300 ℓ-fermenter under the condition as shown in Table 6 below, and the thus cells were harvested by filtration.

The thus recovered cells were uniformly dispersed and suspended in water to give a 12 % suspension.

Next, the resulting suspension was broken with a high-pressure homogenizer (HV-OH-0.7-3.7S Model; manufactured by Izumi Food Machinery Co.) under pressure of 700 kg/cm2, at a flow rate of 60 liters/hr. The broken cells were dewatered with a double drum drier to obtain a dry cell powder having a water content of 3.8 % by weight.

700 g of the dry cell powder was filled into almost the same column as that shown in Fig. 2 (excepting that the former has an inner diameter of 55 mm and a total length of 1400 mm and that it has no cock), and n-hexane of an amount of 4 times the amount of the dry cell fragment powder (or 2.8 liters of n-hexane) was introduced thereinto from the top of the column, whereupon 2.3 liters of a hexane liquid as flown out from the bottom of the column was recovered before the liquid had no yellow color of lipids therewith. This was concentrated under reduced pressure to obtain 215 g of lipidstherefrom. The extraction yield was 94 %.

### EXAMPLES 3 TO 8

Various lipids-producing microorganisms as indicated in Table 7 below were cultured under the condition as indicated in Table 6 below and broken, dried and extracted in the same manner as in Example 1. The extraction yield of each case was shown in Table 7.

### COMPARATIVE EXAMPLE 1

Mucor circinelloides HUT 1121 (FERM BP-3883) were cultured in a 30ℓ-fermenter, and the γ-linolenic acid-containing cells were harvested by filtration.

The thus harvested cells were again dispersed in water to give a 12 % suspension, a part of which was then dried with a double drum dryer to obtain dry cells having a water content of 4 % by weight.

10 g of the dry cells were filled into a column and extracted therethrough in the same manner as in Example 1. As a result, 1.2 g of yellow lipids were obtained. The extraction yield was 31 %.

### COMPARATIVE EXAMPLE 2

300 ml of ethanol was added to 100 g of the wet cells (water content: 65 %) as obtained in Comparative Example 1 and milled in a one-liter ball mill for 5 hours.

After milling, ethanol was removed by filtration under reduced pressure, and the remaining cells were collected. 300 ml of n-hexane was added thereto and again milled in the ball mill for 5 hours for extraction.

By filtration under reduced pressure, the cell residue and n-hexane were separated from each other, and the thus obtained hexane fraction was concentrated under reduced pressure to recover 9.2 g of lipids. The extraction yield was 69 %.

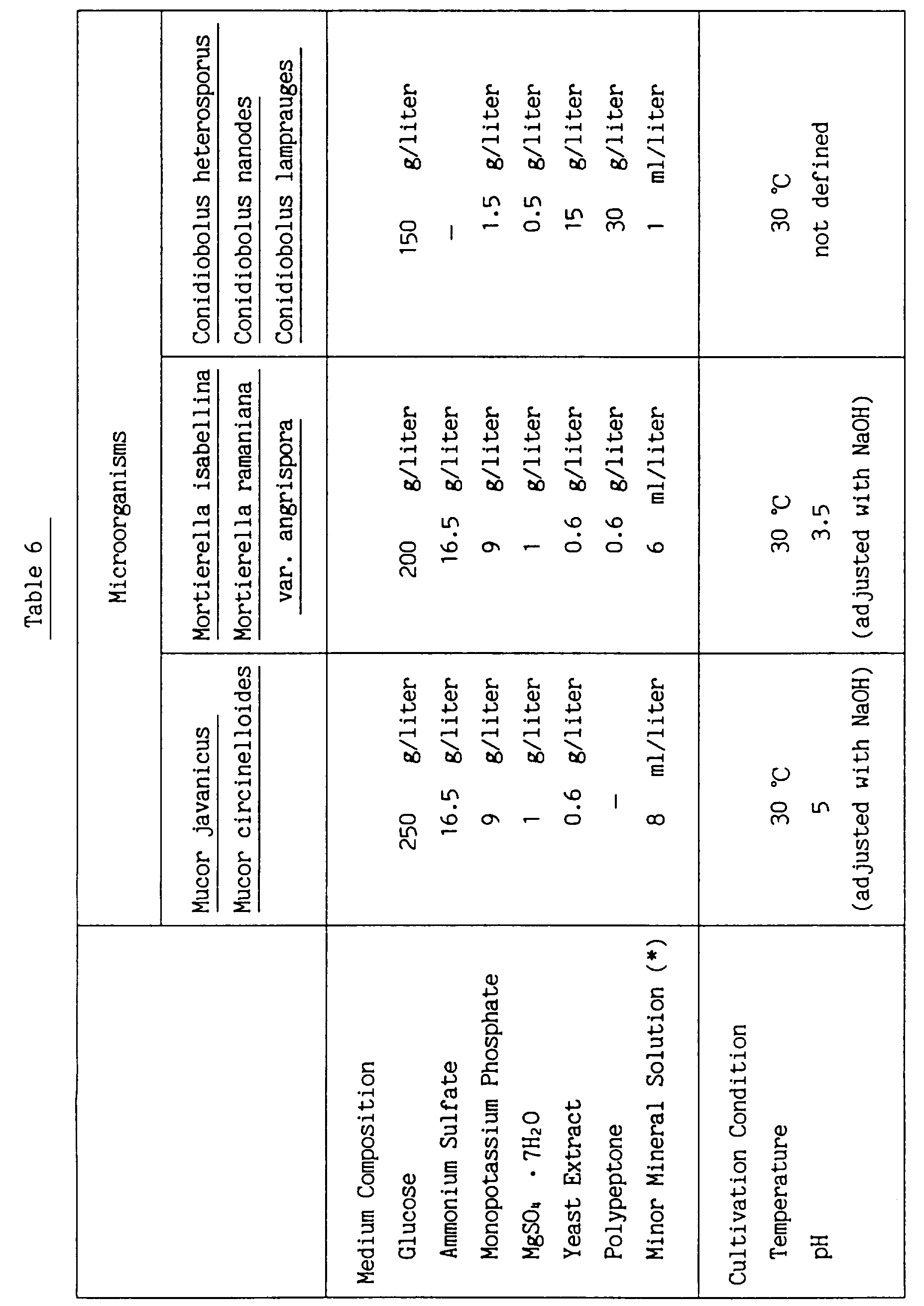

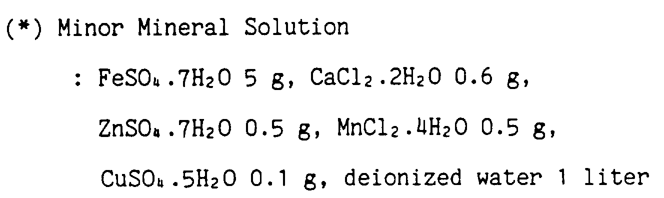

**Table 7**

| Example No. | Microorganisms | Extraction Yield |
|---|---|---|
| 3 | Mortierella isabelliana IFO 7824 | 92 % |
| 4 | Mortierella ramaniana var. angrispora. IFO 8187 | 91 % |
| 5 | Conidiobolus heterosporus ATCC 12941 | 92 % |
| 6 | Conidiobolus nanodes CBS 183/62 ( ATCC 14651 ) | 93 % |
| 7 | Conidiobolus lamprauges ATCC 12585 | 92 % |
| 8 | Mucor javanicus HUT 1162 ( FERM BP-3884 ) | 89 % |

In accordance with the present invention, dry cell fragments of triglyceride-containing microbial cells are obtained by breaking an aqueous dispersion of the cells followed by dewatering it. The dry cell fragments thus obtained by the present invention are characterized by having a decrement of the triglyceride content therein of being not more than 10 % by weight, after heat-treatment of them at 150° C for 2 minutes. Thus, these have excellent storage stability and are stable to heat-treatment in preparing them. Therefore, these are useful for producing feed for animals and pet food, which are much liked by animals and pets.

In addition, the method of the present invention has a high extraction yield and allows lipids (triglycerides) to be extracted from microbial cells extremely efficiently.

Further, the method of the present invention has other industrial advantages than that the amount of the solvent to be used in actually carrying out the method may be small, that the operation of the method is safe and that the process itself of the method is simple.

## Claims

1. A method of preparing triglyceride-containing dry cell fragments in which the decrement of the content of polyunsaturated fatty acids after heat-treatment at 150° C for 2 minutes is not more than 10 % by weight; the method being characterized in that triglyceride-containing microbial cells are broken, while they are dispersed in water, and then dewatered.

2. The method as claimed in claim 1, in which the cells are obtained by cultivating Mucor circinelloides HUT 1121 (FERM BP-3883) or Mucor javanicus HUT 1162 (FERM BP-3884).

3. Triglyceride-containing dry cell fragments in which the decrement of the content of polyunsaturated fatty acids after heat-treatment at 150° C for 2 minutes is not more than 10 % by weight; the cell fragments being obtained by breaking triglyceride-containing microbial cells, while the cells are dispersed in water, followed by dewatering them.

4. Triglyceride-containing dry cell fragments in which the decrement of the content of polyunsaturated fatty acids after heat-treatment at 150° C for 2 minutes is not more than 10 % by weight.

5. A feed composition containing the cell fragments as claimed in claim 4.

6. The feed composition as claimed in claim 5, wherein the cell fragments are incorporated in a proportion of from 0.001 to 100% by weight.

7. A method of recovering triglycerides from triglyceride-containing microbial cells; which is characterized in that triglyceride-containing microbial cells are broken, while they are dispersed in water, then dewatered and extracted with a solvent.

8. The method as claimed in claim 7, in which the extraction with a solvent is effected in a column where the broken and dewatered cell fragments are brought into contact with a solvent therein.

9. The method as claimed in claim 7 or 8, in which the solvent is n-hexane.

## Patentansprüche

1. Verfahren zur Herstellung von Triglycerid-haltigen, trockenen Zellfragmenten, bei dem die Abnahme des Gehaltes an mehrfach ungesättigten Fettsäuren nach einer Hitzebehandlung bei 150°C für 2 Minuten nicht mehr als 10 Gew.-% beträgt, und wobei das Verfahren dadurch gekennzeichnet ist, daß Triglycerid-haltige, mikrobielle Zellen aufgebrochen, wobei die Zellen in Wasser dispergiert sind, und anschließend entwässert werden.

2. Verfahren nach Anspruch 1, bei dem die Zellen durch die Kultivierung von Mucor circinelloides HUT 1121 (FERM BP-3883) oder Mucor javanicus HUR 1162 (FERM BP-3884) erzielt werden.

3. Triglycerid-haltige, trockene Zellfragmente, bei denen die Abnahme des Gehaltes an mehrfach ungesättigten Fettsäuren nach einer Hitzebehandlung bei 150°C für 2 Minuten nicht mehr als 10 Gew.-% beträgt und die durch Aufbrechen von Triglycerid-haltigen, mikrobiellen Zellen, wobei die Zellen in Wasser dispergiert sind, und anschließender Entwässerung erzielt werden.

4. Triglycerid-haltige, trockene Zellfragmente, bei denen die Abnahme des Gehaltes an mehrfach ungesättigten Fettsäuren nach einer Hitzebehandlung bei 150°C für 2 Minuten nicht mehr als 10 Gew.-% beträgt.

5. Futterzusammensetzung, welche die Zellfragmente nach Anspruch 4 enthält.

6. Futterzusammensetzung nach Anspruch 5, worin die Zellfragmente in einem Anteil von 0,001 bis 100 Gew.-% vorliegen.

7. Verfahren zur Rückgewinnung von Triglyceriden aus Triglycerid-haltigen, mikrobiellen Zellen, welches dadurch gekennzeichnet ist, daß Triglycerid-haltige, mikrobielle Zellen aufgebrochen, wobei die Zellen in Wasser dispergiert sind, und anschließend entwässert und mit einem Lösungsmittel extrahiert werden.

8. Verfahren nach Anspruch 7, wobei die Extraktion mit einem Lösungsmittel in einer Säule durchgeführt wird, wo die aufgebrochenen und entwässerten Zellfragmente in Kontakt mit dem Lösungsmittel gebracht werden.

9. Verfahren nach Anspruch 7 oder 8, wobei das Lösungsmittel n-Hexan ist.

## Revendications

1. Méthode de préparation de fragments secs de cellules contenant des triglycérides, dans laquelle la diminution de la teneur en acides gras poly-insaturés après traitement par la chaleur à 150°C pendant 2 minutes n'est pas supérieure à 10 % en poids ; la méthode étant caractérisée en ce que les cellules microbiennes contenant des triglycérides sont cassées, alors qu'elles sont dispersées dans l'eau, puis déshydratées.

2. Méthode revendiquée dans la revendication 1, dans laquelle les cellules sont obtenues en cultivant Mucor circinelloides HUT 1121 (FERM BP-3883) ou Mucor javanicus HUT 1162 (FERM BP-3884).

3. Fragments secs de cellules contenant des triglycérides dans lesquels la diminution de la teneur en acides gras poly-insaturés après traitement par la chaleur à 150°C pendant 2 minutes n'est pas supérieure à 10 % en poids ; les fragments de cellules étant obtenus En cassant des cellules microbiennes contenant des triglycérides, alors que les cellules sont dispersées dans l'eau, suivi par leur déshydratation.

4. Fragments secs de cellules contenant des triglycérides dans lesquels la diminution de la teneur en acides gras poly-insaturés après traitement par la chaleur à 150°C pendant 2 minutes n'est pas supérieure à 10 % en poids.

5. Composition alimentaire contenant les fragments de cellules tels qu'ils sont revendiqués dans la revendication 4.

6. Composition alimentaire telle qu'elle est revendiquée dans la revendication 5, dans laquelle les fragments de cellules sont incorporés dans une proportion allant de 0,001 à 100 % en poids.

7. Méthode de récupération de triglycérides à partir des cellules microbiennes contenant des triglycérides, qui est caractérisée en ce que les cellules microbiennes contenant des triglycérides sont cassées, alors qu'elles sont dispersées dans l'eau, puis déshydratées et extraites avec un solvant.

8. Méthode telle qu'elle est revendiquée dans la revendication 7, dans laquelle l'extraction avec un solvant est effectuée dans une colonne où les fragments de cellules cassées et déshydratées sont mis en contact avec un solvant.

9. Méthode telle qu'elle est revendiquée dans la revendication 7 ou 8, dans laquelle le solvant est le n-hexane.
